# EUROPEAN PATENT APPLICATION

(11) **EP 0 888 770 A1**
(43) Date of publication of application: **07.01.1999**
(21) Application number: 97304914.1
(22) Date of filing: 04.07.1997
(51) Int. Cl.: A61K 9/00, A61K 47/38

(54) **Compositions comprising cationic polysaccharides and cationic drugs**

(71) Applicant: UNION CARBIDE CHEMICALS & PLASTICS TECHNOLOGY CORPORATION, Danbury, Connecticut 06817-0001 (US)
(72) Inventor: Donabedian, David Hagop, Somerset, New Jersey 08873 (US); Marlin, Lawrence, Bridgewater, New Jersey 08807 (US); Eng, Donna Jean, Hartsdale, New York 10530 (US)
(74) Representative: Allard, Susan Joyce

(57) **Abstract**

The present invention relates to delivery systems comprising a cationic polymer, optionally in conjunction with an anionic polymer, to deliver cationic therapeutic agents and their application to a mucosal surface. In particular, these delivery systems are well suited for the treatment of intraocular pressure and glaucoma by way of a sustained delivery system.

## Description

### FIELD OF THE INVENTION

The present invention relates to the use of cationic polymers to deliver cationic therapeutic agents to a mucosal surface, e.g., the eye. The invention also relates to the use of cationic polymers in conjunction with anionic polymers to deliver the cationic therapeutic agents to the mucosal surface.

### BACKGROUND OF THE INVENTION

The delivery of therapeutic agents to mucosal surfaces has inherent difficulties due to the moist nature of the mucosal surfaces. This problem is particularly acute in the delivery of therapeutic agents to the surface of the eye where the washing effects of the tear film removes much of the therapeutic agent. For glaucoma medications, for example, the result is a small pulse of drug is delivered to the target ocular tissues, while the majority of the drug is absorbed systemically.

Accordingly, there is a need for a method for treating mucosal surfaces such as, for example, the eye, in order to retain an active agent for a longer period of time. Moreover, there is a continuing need to produce ophthalmic formulations that are characterized as long lasting (sustained release) and are comfortable to the eye.

### SUMMARY OF THE INVENTION

This invention pertains to topical, substantive delivery systems for mucosal tissues comprising an aqueous solution of a cationic polysaccharide and a cationic therapeutic agent. A preferred delivery system in accordance with the present invention comprises a cationic polysaccharide and an anionic polymer along with a cationic therapeutic agent. The cationic therapeutic agents which can be delivered by the delivery systems of the present invention include, for example, agents known in the art as "basic actives", e.g., timolol and betaxolol and drugs such as pilocarpine, epinephrine, and carbachol. This invention is particularly useful for the treatment of lowering and controlling intraocular pressure ("IOP") in the treatment of glaucoma as well as providing for decreased discomfort as usually associated with the use of such drugs.

### DETAILED DESCRIPTION OF THE INVENTION

Mucosal surfaces in the body include, but are not limited to, the outer covering or globe of the eye, the inside lining of the mouth, nose and vagina. These surfaces are generally soft, moist tissue. For example, the globe or outer covering of the eye is comprised of non-keratinized epithelium (Bloom, W. and Fawcett, D.W., A TEXTBOOK OF HISTOLOGY, 10th Ed., W.B. Saunders Co., Philadelphia 1975). The surface of the eye is continuously coated with water from the tear ducts which frequently washes material away from the outer coating of the eye. It has been surprisingly discovered that the cationic polysaccharide polymers of the present invention are substantive to mucosal surfaces such as the eye while being non-irritating. The mucosal surfaces of the body are not comprised of keratin.

The cationic polysaccharide polymers which are useful in the present invention are those which are substantive to mucosal surfaces and include, but are not limited to: the starch and cellulose families; pectin; chitin; chitosan; guar; and the like. Substantivity of the cationic therapeutic agent and cationic polysaccharide polymer combination is characterized by an increase of the cationic polysaccharide polymer on the mucosal surface. Substantivity can be measured, for example, through the use of an ocular fluorimeter. The cationic polysaccharide polymer is fluorescently tagged by reaction with fluorescein as described in the procedure of De Balder and Granath for labeling dextrans; Carbohydrate Research, 30 (1973) 375-378.

Preferably, the cationic polysaccharides are substituted with greater than about 0.1, preferably from about 0.2 to 1.0, mole per mole of polysaccharide of a quaternary nitrogen compound having hydrocarbon substituents with from 1 to about 4 carbon atoms per substituent. As used herein, the term "substituent" is made with reference to substituents other than the substituent which is connected to the polysaccharides, although it is typical for the connecting substituent to also have up to about 4 carbon atoms. Preferably, the connecting substituent comprises an alkoxyalkyl radical with a least 2 carbon atoms separating the oxygen atom from the nitrogen atom. Preferably, the other substituents will be either methyl or ethyl and the total number of carbon atoms in such other substituents will be from about 3 to 6, more preferably about 4. A preferred regent suitable for substitution onto the polysaccharide is 2,3 epoxypropyl trimethyl ammonium chloride. Preferably, the type of quaternary substitutent and substitution level is effective to provide substantivity to the mucosal surface and a clear solution, even when the solution contains isotonic salt, e.g., 9 grams of sodium chloride per liter of water. Further details concerning the preparation of suitable cationic polysaccharides is known to those skilled in the art.

The molecular weight of the cationic polysaccharides suitable for use in accordance with the present invention typically ranges from about 10,000 to 1,00,000 grams per gram mole and preferably ranges from about 20,000 to 500,000 grams per gram mole. As used herein, the term "molecular weight" means weight average molecular weight. Methods for determining weight average molecular weight of polysaccharides are known to those skilled in the art. One preferred method for determining molecular weight is low angle laser light scattering. The viscosity of the cationic polysaccharides typically ranges from about 5 to 10,000 centipoise, preferably from about 10 to 2,000 centipoise. Unless otherwise indicated, as used herein the term "viscosity" refers to the viscosity of a 2.0 weight percent aqueous solution of the polymer measured at 25°C with a Brookfield viscometer. Such viscosity measuring techniques are known in the art.

Preferred cationic polysaccharides are water soluble, cationic cellulosics which include, but are not limited to, water soluble quaternary nitrogen-containing cellulose ethers such as UCARE® Polymers JR-125, JR-400, JR-30M, LR-400, LR-30M and SR-10 provided by Union Carbide Corporation, Danbury, CT. As used herein, the term "water soluble" is made with reference to a 1 weight percent aqueous solution.

The cationic therapeutic agents suitable for use in accordance with the present invention are those therapeutic agents that bear, or are capable of bearing, a positive charge during formulation or use of the final product. Such base actives are known in the art, see e.g., U. S. Pat. No's. 5,093,126, 4,252,984, and 4,012,444. These materials often have the desired effects of controlling and lowering IOP.

Examples of these cationic therapeutic agents are: beta blockers, including but not limited to, betaxolol, timolol, labetalol, propranolol, bupranolol, befunolol, acebutolol, salbutamol, atenulol, isoxaprolol, esmalol, pindolol, hepunolol, carpranolol, metaprolol, azotinolol, carteolol, diacetolol, and the like; and the following classes of drugs which are used in the treatment of ocular hypertension and glaucoma: epinephrine, pilocarpine, proepinephrine, norepinephrine, pronorepinephrine, clonidine and clonidine derivatives and carbachol. Further, the cationic polymers may have ameliorating properties, thus providing for decreased discomfort usually associated with the use of such therapeutic agents. Such cationic therapeutic agents are commercially available.

In a broad sense, the relative proportion of cationic polymer to cationic therapeutic agent is not narrowly critical. The relative weight ratio of cationic polymer to cationic therapeutic agent will preferably range from 1:1 to about 200:1, more preferably from about 2:1 to about 100:1 and most preferably from about 10:1 to about 50:1.

Typically, the amount of cationic therapeutic agent is at least about 0.0001 weight percent, preferably from about 0.0005 weight percent to about 4.0 weight percent and more preferably from about 0.001 weight percent to about 2.0 weight percent and most preferably from about 0.01 weight percent to less than about 1.0 weight percent based on the total composition.

The amount of cationic polysaccharide polymer provided may also vary widely. In a preferred embodiment the cationic polymer is provided in an amount sufficient to be substantive to the mucosal surface. Typically, the amount of cationic polymer is at least about 0.0005 weight percent, preferably from about 0.0025 weight percent to about 20.0 weight percent and most preferably from about 0.005 weight percent to about 10 weight percent of the total composition.

Through selection and optimization of the various related structural parameters influencing viscosification, cationic polysaccharide polymers of this invention can be produced which provide a desired level of viscosity, within a potentially wide range of values. Aqueous solutions containing 0.5 weight percent concentrations cationic polysaccharide polymers of this invention will usually have a Brookfield viscosity at 25°C of less than 50 centipoise (cps), and preferably from about 5 to about 30 cps.

One aspect of the present invention provides methods for delivery of cationic therapeutic agents to mucosal surfaces with a cationic polysaccharide, and delivery systems comprising the cationic therapeutic agents and the cationic polysaccharide. Without being bound to any particular theory, it is believed that the cationic therapeutic agents, due to it's mobility and size, can easily gain access to the mucosal surface. Once being bound to that surface, either electrostatically or covalently, the cationic polysaccharide can then deposit over the drug in a loop type of conformation and hence entrap it on the surface, thus holding it on the surface longer and increasing drug residence time into the surface. Thus, enhanced effects of sustained drug delivery may be achievable.

Another aspect of the present invention is directed to the use of an anionic polymer that is used to enhance bonding between the cationic polysaccharide and the cationic therapeutic agents. In this aspect of the invention, the anionic polymers include, but are not limited to, glycosaminoglycans, such as, for example, including hyaluronan, hyaluronic acid and it's derivatives, heparin, chondroitin and keratin sulfates. Other anionic polymers include, for example, carboxymethyl cellulose, carboxymethyl starch, carboxymethyl guar and the like, as well as polyacrylic acid and it's derivatives. Such anionic therapeutic agents are commercially available.

The ratio of cationic polymer to anionic polymer can vary. In a preferred embodiment, after the anionic polymer is added to the cationic polymer, sufficient cationic charge remains on the polymer backbone to bind the polymer to the mucosal surface. The relative weight ratio of cationic polymer to anionic polymer can fall in the range of 0.01 to about 200:1, most preferably from 2:1 to about 100:1. The amount of cationic therapeutic agents can also vary in the aqueous solution.

The cationic polymer and cationic therapeutic agents (and anionic polymers when employed) of this invention are typically provided to the mucosal surface in an aqueous solution typically neutral buffered and isotonic, similar to artificial tear solutions. The tonicity or osmolality can be either adjusted to hypotonicity, isotonicity or hypertonicity relative to the normal tear. Such tonicity agents are known in the art but not limited to nonionic agents include dextrose, mannitol glycerin and propylene glycol in amounts varying from 0.0% to 10.0% by weight in the final formulation. Preferably, the range in the level of isotonic salts employed is up to about 0.9 parts by weight for inorganic salts and up to about 6.0 parts by weight for organic substances. Illustrative inorganic isotonicizers include sodium chloride, boric acid and borax, while natural substance isotonicizers are generally sugars such as mannitol and sorbital. The pH of these isotonicized solutions can vary widely from 3 to 9. When the delivery systems are for use in the eye, the pH of the solution should be as close as possible to neutral and should always be within the range of pH 6-8.

Preservatives are commonly employed in most multi dosage ophthalmic formulations to prevent microbial contamination during use. Suitable preservatives include benzalkonium chloride, POLYQUAD®, thimerosal, edetate disodium, chlorobutanol and the like. Typically, such preservatives are used in levels ranging from 0.001% to 1.0% by weight in the final composition. Thickeners are often added to ophthalmic preparations to produce desirable viscosities depending upon application. Common viscosifiers include hydroxyethyl cellulose, hydroxypropylmethyl cellulose, polyvinyl alcohol, polyvinyl pyrolidone, dextrose, and the like. The selection and amount of other optional ingredients contained in the compositions of this invention are not critical but will vary depending upon the particular ingredient, composition and desired use level and may be any effective amount for achieving the desired property provided by such ingredients, following procedures known to those in the art.

Preferably, the solutions containing the cationic polysaccharides, cationic therapeutic agents and anionic polymers, when employed, are clear, i.e., not cloudy so as to cause visual distortion or other visual problems.

The aqueous solutions of the present invention can be provided topically to the mucosal surface as a gel, lotion or cream. In a preferred embodiment , drops are used to deliver the current formulation.

The compositions of the present invention are useful, for example, in the treatment of lowering intraocular pressure and glaucoma.

The following specific examples illustrate certain aspects of the present invention and are set forth for illustration only and are not to be construed as limitations on the appended claims. All parts and percentages are by weight unless otherwise specified.

### EXAMPLES

The following examples are representative of delivery systems in accordance with the present invention useful in the sustained release of cationic therapeutic agents for lowering and controlling IOP and glaucoma. These formulations also provide for amelioration of harsh therapeutic agents.

### DEFINITIONS

The following designations used in the Examples and elsewhere herein have the following meaning:
- JR -: UCARE® Polymer JR 400, a cationic cellulosic polymer (N,N,N-trimethyl ammonium chloride hydroxyethyl cellulose) with a molecular weight of approximately 500,000 available from Union Carbide Corporation, Danbury, CT.
- Na Hyaluronic Acid -: Glycosaminoglycan with a molecular weight of approximately 2,000,000 available from Genzyme Corporation, Cambridge, MA.

### EXAMPLE 1

A 0.55% solution of purified JR and Na Hyaluronic Acid were prepared in a balanced salt solution at a weight ratio of 10:1. The pH of the solution was 7.4. To this clear and colorless solution, 3.3 g of mannitol was added followed by 0.25 g of carbachol. Benzalkonium chloride (0.005%) was added as a preservative and the contents were allowed to mix for 1 hour. The contents remained clear and colorless with a pH = 7.4 and were filter sterilized through a 0.22 µ cartridge at 25 °C. The final solution viscosity was 27.7 dl/g.

### EXAMPLE 2

A 0.5% solution of JR and Na Hyaluronic Acid were prepared in a balanced salt solution at a weight ratio of 10:1. The pH of the solution was 7.2. To this clear and colorless solution, 1.0 mL of a 10 % aqueous pilocarpine solution was added. The contents were allowed to mix for several hours. The contents remained clear and colorless. The pH of the final solution was 6.0.

### EXAMPLE 3

A 0.5% solution of JR and Na Hyaluronic Acid were prepared in a balanced salt solution at a weight ratio of 10:1. The pH of the solution was 7.2. To this clear and colorless solution, 1 mL of a 10% aqueous pilocarpine solution was added. and the contents were allowed to mix for several hours. The contents remained clear however were no longer colorless. Associated with the epinephrine solution was a slight brownish color.

### EXAMPLE 4

A 0.5% solution of JR and Na Hyaluronic Acid were prepared in a balanced salt solution at a weight ratio of 2:1. The pH of the solution was 7.2. To this clear and colorless solution, 9 mL of a 10% aqueous carbachol solution was added and the contents were allowed to mix for several hours. The drug content was 1.5%. The contents remained clear and colorless with a pH = 7.4.

### EXAMPLE 5

A 0.5% solution of JR and Na Hyaluronic Acid were prepared in a balanced salt solution at a weight ratio of 10:1. The pH of the solution was 7.2. To this clear and colorless solution, 2.0 mL of a 10% aqueous carbachol solution was added. and the contents were allowed to mix for several hours. The contents remained clear and colorless with a pH = 7.4.

### EXAMPLE 6

A 0.5% solution of purified JR and Na Hyaluronic Acid were prepared in a balanced salt solution at a weight ratio of 5:1. The pH of the solution was 7.2. To this clear and colorless solution, 5.0 mL of a 10 % aqueous pilocarpine solution was added. The contents were allowed to mix for several hours. The contents remained clear and colorless. The pH of the final solution was 6.0.

## Claims

1. A method for delivery of a cationic therapeutic agent to a mucosal surface, which method comprises providing a solution to said mucosal surface, said solution comprising:
(a) water;
(b) an effective amount of a cationic polysaccharide polymer substituted with greater than 0.1 moles per mole of polysaccharide of a quaternary nitrogen compound having hydrocarbon substituents with from 1 to 4 carbon atoms per substituent to provide substantivity to the mucosal surface and a clear solution; and
(c) an effective amount of a cationic therapeutic agent to provide delivery of the cationic therapeutic agent to the mucosal surface and retain said clear solution.

2. A method as claimed in claim 1 wherein the cationic polysaccharide polymer is a cationic cellulosic.

3. A method as claimed in claim 1 or claim 2 wherein the cationic therapeutic agent is betaxolol, timolol, labetalol, propranolol, bupranolol, befunolol, acebutolol, salbutamol, atenulol, isoxaprolol, esmalol, pindolol, hepunolol, carpranolol, metaprolol, azotinolol, carteolol, diacetolol, epinephrine, pilocarpine, proepinephrine, norepinephrine, pronorepinephrine, clonidine or clonidine derivatives, carbachol or mixtures thereof.

4. A method as claimed in any one of the preceding claims wherein the solution further comprises an effective amount of an anionic polymer to enhance the delivery of the cationic therapeutic agent to the mucosal surface and retain said clear solution.

5. A method as claimed in claim 4 wherein the anionic polymer is hyaluronan, hyaluronic acid, a hyaluronan derivative, carboxymethyl cellulose, carboxymethyl starch, carboxymethyl chitin, chitosan, guar, poly acrylic acid or a derivative thereof, or mixtures thereof.

6. A sustained release topical composition comprising:
(a) a cationic therapeutic agent:
(b) an effective amount of a cationic polysaccharide polymer substituted with greater than 0.1 moles per mole of polysaccharide of a quaternary nitrogen compound having hydrocarbon substituents with from 1 to 4 carbon atoms per substituent to provide substantivity to the mucosal surface and a clear solution; and
(c) water.

7. A composition as claimed in claim 6 further comprising (d) an effective amount of an anionic polymer to enhance the delivery of the cationic therapeutic agent to the mucosal surface and retain said clear solution.

8. A composition of claim 6 wherein the anionic polymer is selected from the group consisting of hyaluronan, hyaluronic acid, a hyaluronan derivative, carboxy methyl cellulose, carboxymethyl starch, carboxymethyl chitin, chitosan, guar, a poly acrylic acid or a derivative thereof, or mixtures thereof.

9. A composition as claimed in claim 7 wherein the concentration of the anionic polymer is from 0.001 to 0.6 wt % based on the total weight of the composition.

10. A composition as claimed in any one of claims 6 to 10 wherein the concentration of the cationic therapeutic agent is from 0.001 to 0.5 wt % based on the total weight of the composition.
